# EUROPEAN PATENT SPECIFICATION

(11) **EP 0 433 011 B1**
(45) Date of publication and mention of the grant of the patent: **20.07.1994**
(21) Application number: 90313433.6
(22) Date of filing: 11.12.1990
(51) Int. Cl.: A61F 2/06, A61N 5/10

(54) **Intra-arterial stent with the capability to inhibit intimal hyperplasia**
Intraarterielle Stützeinrichtung mit der Fähigkeit der Hemmung einer Intimahyperplasie
Dilateur intra-artériel capable d'inhiber une hyperplasie de l'intima

(30) Priority: 11.12.1989 US 448691
(43) Date of publication of application: 19.06.1991
(60) Divisional application: 93203354.1
(73) Proprietor: Fischell, Robert E., Dayton, Maryland 21036 (US); FISCHELL, Tim A., Los Altos, CA 94022 (US)
(72) Inventor: Fischell, Robert E., Dayton, Maryland 21036 (US); FISCHELL, Tim A., Los Altos, CA 94022 (US)
(74) Representative: Lambert, Hugh Richmond

(56) References cited:
- DE-C- 867 433
- US-A- 1 954 868
- US-A- 3 351 049
- US-A- 4 768 507

## Description

This invention relates to intra-arterial stents that are used to maintain patency of an arterial lumen typically subsequent to balloon angioplasty or atherectomy.

Since the mid-to late 1980's, intra-arterial stents have found extensive use as a treatment to prevent restenosis subsequent to balloon angioplasty or atherectomy. A recurrent problem is that excessive tissue growth (intimal hyperplasia) at the site of the balloon dilation or atherectomy plaque excision results in restenosis of the artery. One possible solution to this problem (US Patent No. 4,768,507) is to coat the stent with an anti-thrombogenic surface so as to reduce platelet fibrin deposition. Although an anti-thrombogenic coating can prevent acute thrombotic arterial closure and decrease the need for anticoagulant drug therapy, there is still an urgent need to decrease restenosis which is caused by intimal hyperplasia.

It is well known that radiation therapy can reduce the proliferation of rapidly growing cancer cells in a malignant tumour, and this is made use in the present invention, which resides in a stent comprising a tubular structure insertable into an artery and locatable therein to maintain the lumen of the artery patent, wherein the stent comprises or is constructed of a material that is radioactive. Preferably the stent comprises a helical wire spring of the type shown in US Patent 4,768,507 and which either incorporates or is coated with a radioactive isotope, preferably a beta emitter.

Tubular radioactive structures for insertion into the body are, of course, known. For example, US-A-3,351,049 discloses a radioactive seed for radiation therapy comprising an elongate stainless steel tube, sealed at both ends and having sealed therein a radioactive source, e.g. a filamentous nylon body impregnated with a soft X-ray emitter such as I¹²⁵. Such seeds are simply used as a radioactive implant to be implanted in a tumour to provide a highly localised source of radioactivity.

An even more basic radioactive seed is disclosed in US-A-1954868 dating from 1929. This simply consists of a single wall capillary tube sealed at both ends and containing a quantity of radon as the radioactive source. A similar seed is shown in DE-C-867433. In this case the seed comprises a tube of a radioactive isotope sealed in a carrier tube, e.g. of Monel metal, glass, ceramic or graphite. Such devices are of little relevance to the object of the present invention, i.e. the prevention of restenosis in an artery following balloon angioplasty or atherectomy.

This invention is further described with reference to the accompanying drawings in which:
Figure 1 is a cross-section showing two turns of a radioisotope helical coil spring stent embedded into a balloon dilated or atherectomized plaque within a human artery;
Figure 2 is a cross-section through the spring wire of a helical coil spring stent showing a radioisotope core material within the spring material;
Figure 3 is a cross-section through the spring wire of a helical coil spring stent showing a thin plating or radioisotope material on the exterior surface;
Figure 4 is a cross-section through a central core spring wire of a helical coil spring stent showing a radioisotope plating which is covered with an anti-thrombogenic coating.

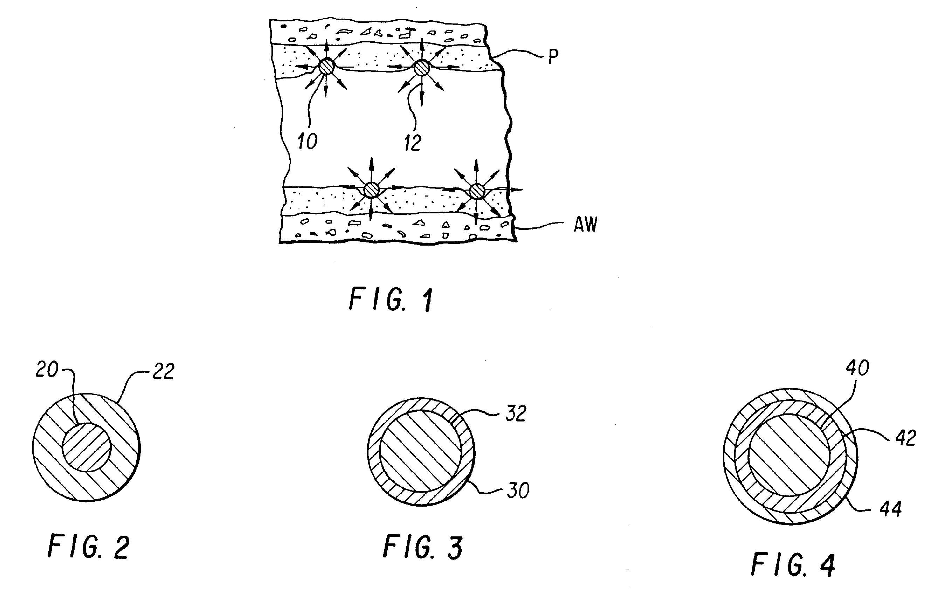

Referring to the drawings, the present invention employs intra-arterial stents of the type shown in US Patent No. 4,768,507 and which comprise a helical coil spring typical cross-sections of which are shown in Figures 5 and 6 of that patent. Two turns of such a helical coil spring stent (10) fabricated from a pure metal or alloy are shown in Figure 1 of the present drawings as they would be disposed in use imbedded into plaque (P) within the arterial wall of the artery (AW). In accordance with the present, wire stent is irradiated so that it has become radioactive, i.e. it now comprises a radioisotope, and the arrows (12) pointing outward from the cross-section (10) indicated the omnidirectional emission of particles from the stent wire. The purpose of this radiation is to decrease the rate of proliferative cell growth of the traumatized arterial wall AW (which growth is termed "intimal hyperplasia"). Thus it would be expected that restenosis, which frequently occurs after stent implantation, will be significantly reduced.

The radioisotope used for this purpose may be alpha, beta or gamma emitter. The half-life would ideally be between 10 hours and 100 days. An optimum emitter might be a beta emitting isotope such as vanadium 48 which has a half-life of 16 days and only 8% of its emitted energy is from gamma radiation. The ideal attribute of a beta emitter is that the radiation does not travel very far in human tissue. Thus only the tissue in close proximity to the radioisotope stent will be affected. Furthermore only moderate levels of radiation are desired since it is known that very high levels can cause injury to non-proliferating tissues.

Another method to make the material of the stent spring wire is from a metal into which is alloyed an element that can be made into a radioisotope. For example, phosphorus 32, a 14.3 day half-life beta emitter, could be alloyed into steel which could be used for the stent wire.

Figure 2 shows a stent wire cross-section in which a wire made from a radioisotope core material 20 is formed within an outer covering 22 that has the attributes that are desirable for being a coil spring stent.

Figure 3 shows a cross-section of an alternative embodiment of the present invention in which a radioisotope coating 30 is plated onto a spring material core 32. For example, the beta emitting isotope gold 198 (half-life 2.7 days) could be used to coat any suitable spring metal material.

Figure 4 shows a more complex stent cross-section in which a core of some material ideally suited for stents is plated with a radioisotope coating 42 which is, in turn, coated with an anti-thrombogenic coating 42 such as carbon as described in US Patent No. 4,768,507.

Although helical coil spring stents have generally been described herein, the concept of utilizing a radioactive material within the stent structure so as to attenuate intimal hyperplasia is certainly applicable to any stent design. Furthermore, the temporary placement at the site of the vessel wall trauma of a radioactive source with the arterial lumen, for example a thin wire with a radioactive tip which wire can be withdrawn after a limited time is also envisioned.

Various other modifications, adaptations and alternative designs are of course possible in light of the above teachings without departing from the scope of the invention as herein described and hereinafter claimed.

## Claims

1. For use in the prevention of restenosis of an artery following balloon angioplasty or atherectomy and resulting from intimal hyperplasia, a radially expansible intra-arterial stent (10) comprising a radially expansible, generally tubular structure insertable into the artery and automatically expansible therein from a first diameter enabling the stent to be introduced into the artery to a second, larger diameter in which the stent engages the arterial wall to maintain the arterial lumen patent, said generally tubular structure itself having a longitudinal opening extending therethrough for the throughflow of arterial blood through the stent, characterized in that the said stent comprises a radioactive isotope effective, when the expanded stent is engaged within the artery, to prevent restenosis of the artery in the vicinity of the stent.

2. A stent according to claim 1, characterised in that the said radioisotope is incorporated into the material of the stent.

3. A stent according to claim 1, characterised in that said radioisotope is plated onto the surface of the stent.

4. A stent according to any one of claims 1 to 3, characterised in that the said radioisotope is a beta-particle emitter.

5. A stent according to any one of claims 1 to 4, characterised in that the said radioisotope has a half-life of less than 100 days.

6. A stent according to claim 5, characterised in that the radioisotope is vanadium 48 or gold 198.

7. A stent according to any one of claims 1 to 6, characterised in that the stent is coated with anti-thrombogenic material.

8. A stent according to any one of claims 1 to 7, which is in the form of a radioactive helical wire spring.

## Patentansprüche

1. Radial ausdehnbare intraarterielle Stützeinrichtung (10) für die Verwendung bei der Verhinderung von Restenose einer Arterie nach Ballonangioplastie oder Atherektomie, die aus Intimahyperplasie resultiert, mit einer radial ausdehnbaren, allgemein röhrenförmigen Struktur, die in die Arterie einführbar und darin von einem ersten Durchmesser, der die Einführung der Stützeinrichtung in die Arterie ermöglicht, automatisch zu einem zweiten, größeren Durchmesser, mit welchem die Stützeinrichtung an der Arterienwand anliegt, um das Arterienlumen offenzuhalten, ausdehnbar ist, wobei die allgemein röhrenförmige Struktur selbst eine Längsöffnung hat, die sich für den Durchfluß von Arterienblut durch die Stützeinrichtung durch diese hindurch erstreckt, **dadurch gekennzeichnet,** daß die Stützeinrichtung ein radioaktives Isotop umfaßt, welches, wenn sich die ausgedehnte Stützeinrichtung in der Arterie in Anlage an ihr befindet, wirksam ist, Restenose der Arterie in der Nachbarschaft der Stützeinrichtung zu verhindern.

2. Stützeinrichtung nach Anspruch 1, **dadurch gekennzeichnet,** daß das Radioisotop in das Material der Stützeinrichtung eingearbeitet ist.

3. Stützeinrichtung nach Anspruch 1, **dadurch gekennzeichnet,** daß das Radioisotop als Überzug auf der Oberfläche der Stützeinrichtung aufgebracht ist.

4. Stützeinrichtung nach einem der Ansprüche 1 bis 3, **dadurch** **gekennzeichnet,** daß das Radioisotop ein beta-Teilchenemitter ist.

5. Stützeinrichtung nach einem der Ansprüche 1 bis 4, **dadurch** **gekennzeichnet,** daß das Radioisotop eine Halbwertszeit von weniger als 100 Tagen hat.

6. Stützeinrichtung nach Anspruch 5, **dadurch gekennzeichnet,** daß das Radioisotop Vanadin 48 oder Gold 198 ist.

7. Stützeinrichtung nach einem der Ansprüche 1 bis 6, **dadurch** **gekennzeichnet,** daß die Stützeinrichtung mit einem antithrombogenen Material überzogen ist.

8. Stützeinrichtung nach einem der Ansprüche 1 bis 7, die in der Form einer radioaktiven Spiralwicklungsfeder vorliegt.

## Revendications

1. Pour utilisation dans la prévention de la resténosis d'une artère à la suite de l'angioplastie par poches et l'athérectomie et résultant de l'hyperplasie de l'intima, un dilatateur (10) intra-artérielle radialement extensible comprenant une structure généralement tubulaire extensible radialement insérable dans l'artère et automatiquement extensible dans celle-ci à partir d'un premier diamètre permettant au dilatateur de s'introduire dans l'artère à un second diamètre plus grand grâce à quoi le dilatateur s'engage dans la paroi artérielle pour maintenir non obstruée la lumière artérielle, ladite structure généralement tubulaire elle-même ayant une ouverture longitudinale s'étendant à travers celle-ci pour l'écoulement du sang artériel à travers le dilatateur, caractérisé en ce que ledit dilatateur comprend un isotope radioactif efficace, lorsque le dilatateur en expansion s'est engagé à l'intérieur pour empêcher la resténosis de l'artère au voisinage du dilatateur.

2. Un dilatateur selon la revendication 1, caractérisé en ce que ledit radio-isotope est incorporé dans le matériau du dilatateur.

3. Un dilatateur selon la revendication 1, caractérisé en ce que ledit radio-isotope est plaqué sur la surface du dilatateur.

4. Un dilatateur selon une quelconque des revendications 1 à 3, caractérisé en ce que ledit radio-isotope est un émetteur de particules bêta.

5. Un dilatateur selon une quelconque des revendications 1 à 4, caractérisé en ce que ledit radio-isotope a une demi-vie de moins de 100 jours.

6. Un dilatateur selon le revendication 5, caractérisé en ce que le radio-isotope est le vanadium 48 ou l'or 198.

7. Un dilatateur selon une quelconque des revendications 1 à 6, caractérisé en ce que le dilatateur est revêtu d'un matériau anti-thrombogénique.

8. Un dilatateur selon une quelconque des revendications 1 à 7, qui est sous la forme d'un ressort de fil hélicoïdal radioactif.
